Europäisches Patentamt

European Patent Office

Office européen des brevets

(19)

(11) Veröffentlichungsnummer: **0 182 795 B1**

## (12) EUROPÄISCHE PATENTSCHRIFT

(45) Veröffentlichungstag der Patentschrift: **29.07.92**

(51) Int. Cl.⁵: **G01N 27/22**, G01N 33/28

(21) Anmeldenummer: **85901380.7**

(22) Anmeldetag: **09.04.85**

(86) Internationale Anmeldenummer:
**PCT/DE85/00107**

(87) Internationale Veröffentlichungsnummer:
**WO 85/04718 (24.10.85 85/23)**

(54) MESSFÜHLER ZUR UNTERSUCHUNG VON FLÜSSIGKEITEN.

(30) Priorität: **06.04.84 DE 3413135**

(43) Veröffentlichungstag der Anmeldung:
**04.06.86 Patentblatt 86/23**

(45) Bekanntmachung des Hinweises auf die
Patenterteilung:
**29.07.92 Patentblatt 92/31**

(84) Benannte Vertragsstaaten:
**AT BE CH DE FR GB LI LU NL SE**

(56) Entgegenhaltungen:
**EP-A- 0 058 102**
**EP-A- 0 082 934**
**DE-B- 2 938 434**
**GB-A- 2 137 361**
**US-A- 3 453 143**

**Measurement Techniques, Band 17, No. 10,
März 1975, New York (US) S.D. Benin et al.:
"A frequency dielectric method of determining salt contents on crude petroleum and
petroleum products", Seiten 1589-1592, sie-**
he Seite 1590, Abschnitt 1

(73) Patentinhaber: **FRAUNHOFER-GESELLSCHAFT
ZUR FÖRDERUNG DER ANGEWANDTEN FOR-
SCHUNG E.V.
Leonrodstrasse 54
W-8000 München 19(DE)**

(72) Erfinder: **HELLWIG, Günter
Im Langen Hau 29
W-7000 Stuttgart 29(DE)**
Erfinder: **BAUSEER, Herbert
Bärenseestr. 8
W-7000 Stuttgart 80(DE)**
Erfinder: **CHMIEL, Horst
Faracelsusstr. 14
W-7250 Leonberg(DE)**

(74) Vertreter: **Schiller, Walter, Dr. et al
Kanzlei Münich, Steinmann, Schiller Willibaldstrasse 36-38
W-8000 München 21(DE)**

**Beschreibung**

**Technisches Gebiet**

Die Erfindung betrifft einen Meßfühler zur Untersuchung von Flüssigkeiten gemäß dem Oberbegriff des Patentanspruchs 1, die Verwendung dieses Meßfühlers sowie eine Vorrichtung zum Messen von Eigenschaften einer Flüssigkeit mittels dieses Meßfühlers.

In der Vergangenheit ist auf den verschiedensten Wegen versucht worden, Meßfühler zur kontinuierlichen und diskontinuierlichen Untersuchung von Eigenschaften von Flüssigkeiten zu realisieren.

So sollen beispielsweise üblicherweise über Meßfühler pH-Wert-Schwankungen in Reaktoren untersucht oder auch physiologische pH-Werte in Blutproben und ähnlichem bestimmt werden. Gegenüber optischen Verfahren, beispielsweise spektroskopischen Verfahren, die ebenfalls kontinuierlich oder diskontinuierlich eingesetzt werden können, haben Meßfühler den Vorteil, daß sie von Trübungen oder die Lichtdurchlässigkeit beeinflußenden Verunreinigungen unabhängig sind und direkt in Proben eingesetzt werden können.

**Stand der Technik**

Ein Meßfühler zur Untersuchung von Flüssigkeiten mit zwei Elektroden ist beispielsweise aus der DE-B-29 38 434 bekannt. Dieser kapazitive Meßfühler weist eine zwischen den Elektroden angeordnete nichtleitende Membran auf, die so ausgebildet ist, daß in sie die Feuchtigkeit eindringen kann. Dabei lassen die im Anspruch 2 der DE-B-29 38 434 angegebenen Membranmaterialien erkennen, daß der "Eindringvorgang" ein Diffusionsvorgang sein soll. Diffusionsvorgänge haben jedoch den Nachteil, daß die Einstellung eines Gleichgewichts zwischen der den Meßfühler umgebenden Flüssigkeit und der "im Membranmaterial befindlichen" Flüssigkeit sehr lange dauert, so daß kurzfristig auftretende Veränderungen erst spät erkannt werden können. Damit ist ein derartiger Meßfühler beispielsweise zur Untersuchung von Alterungsvorgängen in Ölen nur bedingt geeignet.

Darüberhinaus kann die im Membranmaterial befindliche Flüssigkeit unter Umständen die Elektroden "kurzschließen", da diese gegen das Membranmaterial nicht isoliert sind.

Meßfühler zur Untersuchung von Flüssigkeiten sind weiterhin aus der deutschen Offenlegungsschrift 21 55 568 oder den europäischen Offenlegungsschriften EP-A-0 058 102 und EP-A-0 082 934 bekannt. Beispielsweise bei der in der EP-A 0 058 102 beschriebenen Anordnung sind die Elektroden durch ein Oxyd-Material isoliert.

Auch in der DE-OS 21 55 568 ist ein als Kondensator ausgebildeter Meßfühler beschrieben, bei dem eine Kunststoffolie aus in Wasser quellendem Material als Dielektrikum des Kondensators dient, dessen Kapazität ein Maß für die Konzentration von Wasser bzw. darin gelöster Komponenten ist. Der aus der DE-OS 21 55 568 bekannte Meßfühler hat den Nachteil, daß in Wasser quellende Kunststoffmembrane je nach ihrer Aufbewahrung bzw. ihrem Alter unterschiedliche physikalische Eigenschaften zeigen, so daß eine häufige Nacheichung derartiger Fühler notwendig ist. Die in Wasser quellfähigen Kunststoffe sind darüberhinaus in aggressiven Medien oder auch in Mischungen Wasser /organische Lösungsmittel schlecht oder überhaupt nicht einsetzbar.

Aus der DE-PS 925 621 ist ferner ein Meßfühler bekannt, bei dem ein Kondensator mit isolierten Elektroden zur Bestimmung der Dielektrizitätskonstante eines zwischen den Kondensatorelektroden eingebrachten gasförmigen Mediums verwendet wird; ferner wird die Kapazität des Kondensators in Abhängigkeit vom eingebrachten Medium gemessen. Durch diese Meßsonde ist es möglich, den Taupunkt des zu untersuchenden gasförmigen Mediums zu bestimmen.

Aus "Technisches Messen tm, 1979, Heft 6, Seiten 255-259", letztlich ist ein Verfahren zur Bestimmung der komplexen Dielektrizitätskonstanten von Flüssigkeiten mit sehr hoher Energieaufnahme im dm-Wellenbereich beschrieben, wobei die zu untersuchende Probe in einem Koaxialresonator eingeschlossen und die Resonanzfrequenzverschiebung und Güteänderung des Resonanzsystems unter dem Einfluß des zu untersuchenden Materials im Resonator bestimmt wird.

Der dort beschriebene Koaxialresonator erfordert das Einbringen der Flüssigkeit in den Resonator-Hohlraum, so daß lediglich eine diskontinuierliche und keine kontinuierliche Messung möglich ist. Der Aufbau eines derartigen Resonators erfordert zudem erheblichen technischen Aufwand - schon wegen der Abdichtung - so daß es sich hierbei nicht um eine robuste, vielseitig anwendbare Sonde handelt, die auch für kontinuierlichen Betrieb, beispielsweise in Bioreaktoren, zur Messung von Motorölen beim Betrieb und ähnlichem einsetzbar ist.

Den bekannten Meßfühlern, bei denen eine Membran verwendet wird, in die die zu untersuchende Flüssigkeit eindringen kann, ist gemeinsam, daß der Austausch zwischen der in die Membran eingedrungenen Flüssigkeit und der den Meßfühler umgebenden Flüssigkeit nur verhältnismäßig langsam vonstatten geht, da - wie erfindungsgemäß erkannt worden ist - der zugrundeliegende Vorgang ein Diffusionsvorgang ist.

## Beschreibung der Erfindung

Es ist daher Aufgabe der Erfindung, einen Meßfühler anzugeben, der einfach und robust im Aufbau und zum diskontinuierlichen und/oder kontinuierlichen Meßbetrieb in Flüssigkeiten einsetzbar ist, und der vergleichsweise schnell auf Änderungen der Zusammensetzung der Flüssigkeit anspricht.

Es ist weiterhin Ziel der Erfindung, einen Meßfühler zu schaffen, dessen Abmessungen so klein sind, daß er ohne Schwierigkeiten in kleinsten Flüssigkeitsvolumina einsetzbar ist.

Der erfindungsgemäße Meßfühler soll darüberhinaus zur Erfassung verschiedener Flüssigkeitseigenschaften geeignet, also vielseitig verwendbar sein.

Ferner soll eine Vorrichtung angegeben werden, die in Verbindung mit dem erfindungsgemäßen Meßfühler eine quantitative Messung von Flüssigkeitseigenschaften gestattet.

Eine erfindungsgemäße Lösung dieser Aufgabe sowie der vorstehend genannten sonstigen Ziele ist mit ihren Weiterbildungen in den Patentansprüchen gekennzeichnet.

Erfindungsgemäß weisen die Elektroden einen elektrisch isolierenden Überzug auf, der flüssigkeitsdicht ist. Die zu untersuchende Flüssigkeit kann damit lediglich in die Membran eindringen, die hierzu durchgängige Poren, Spalte oder Zwischenräume mit einer großen Oberfläche, beispielsweise in der Größenordnung von $100 m^2/g$ aufweist, so daß sich durch physikalische Adsorption eine Raumladungsschicht ergibt.

In den Poren etc. bilden sich Oberflächenladungen zwischen den Öleinschlüssen und dem Isolator, aus dem die Membran besteht. Diese Oberflächenladungen beeinflußen nicht nur die Dielektrizitätskonstante des Kondensators in Abhängigkeit beispielsweise von der Alkalität des Öls sowie der Dispergenz/Detergenz-Additivkonzentration; die Beweglichkeit der Oberflächenladungen, die über dynamische Messungen erfaßt wird, ist auch eine Maß für die Viskosität des Öls, so daß mit dem erfindungsgemäßen Meßfühler überraschenderweise auch die Viskosität sehr genau gemessen werden kann. Der erfindungsgemäße Meßfühler ist damit hervorragend zur Messung von Eigenschaften insbesondere technischer Öle geeignet, wie nachstehend noch im einzelnen erläutert werden wird.

Ferner ist es auch möglich, als Membranmaterial ein Material zu verwenden, das keine Poren im vorstehend erläuterten Sinne aufweist, sondern das eigentlich flüssigkeitsdicht wäre, bei dem eine "Porenfunktion" jedoch durch Spalte, Zwischenräume etc. realisiert wird, die sich beim (fixierten) Aufeinanderlegen von Schichten aus Membranmaterial oder durch Aufeinanderlegen der beiden isolierten Elektroden ergeben. Dabei kann unter Umständen auf die Membran im vorstehend erläuterten Sinne verzichtet werden, da der Spalt zwischen den Überzügen als Membran dient.

Es kann jedoch nicht nur bei der Messung in Ölen, sondern auch beispielsweise bei der Messung von Ionenaktivitäten und insbesondere des pH-Werts vorteilhaft sein, wenn die "spezifische Porenoberfläche" möglichst groß ist, beispielsweise in der Größenordnung von $100 m^2/g$.

In jedem Falle wird der komplexe Widerstand des von den Elektroden gebildeten Kondensators, zwischen denen sich ein von dem Überzug der einen Elektrode, der Membran und dem Überzug der anderen Elektrode gebildetes Dielektrikum befindet, nur durch die in der Membran bzw. in den Poren, Spalten oder Zwischenräumen der Membran "befindliche" Flüssigkeit verändert, nicht jedoch beispielsweise durch Ohm'sche Anteile einer durchgehenden galvanischen Verbindung, wie sie sich beispielsweise durch einen Flüssigkeitsfaden zwischen den Elektroden ergeben würde.

Da ferner die Membran aus einem Material besteht, das von der zu untersuchenden Flüssigkeit chemisch nicht angegriffen wird, ist die Reproduzierbarkeit von Messungen mit dem erfindungsgemäßen Meßfühler hoch, so daß die Änderung der komplexen Dielektrizitätszahl der Membran bei Eindringen von Flüssigkeit in diese bzw. der komplexen Kapazität des durch die Elektroden gebildeten Kondensators mit überraschend großer Genauigkeit dazu verwendet werden kann, um Eigenschaftsänderungen von Flüssigkeiten zu untersuchen.

Dabei ist es besonders vorteilhaft, wenn die Elektroden im wesentlichen aus einem Metall und/oder Übergangsmetall und insbesondere aus Aluminium, Zirkon, Titan, Tantal, Wolfram oder Molybdän bestehen (Anspruch 2), wobei eine Herstellung der Elektroden aus Reintitan oder einer Titanlegierung, beispielsweise mit einem Gehalt von bis zu 6% Aluminium und bis zu 4% Vanadin und gegebenenfalls Kupfer gemäß Anspruch 8 besonders bevorzugt ist.

Die nichtleitende Membran, die das zu untersuchende Medium aufnimmt oder mit ihm in Berührung ist, besteht gemäß Anspruch 3 bevorzugt aus Aluminiumoxid, Siliziumnitrid, $[KAl_2(OH)_2]^*[AlSi_3O_{10}]$, Titanoxid oder Polytetrafluorethylen.

In Anspruch 4 folgende sind vorteilhafte Materialien für die Elektrodenüberzüge angegeben. Diese bestehen vorteilhafterweise aus einem nichtleitenden Metalloxid, Metallmischoxid, Metallnitrid, einem anorganischen Isolationsmaterial oder Fluorcarbon, besonders bevorzugt aus Oxiden, wie $Al_2O_3$ oder Titanoxid oder Mischoxiden, wie Titan-Aluminiumzirkonat. Die Überzüge können durch direkte Oxidation des Elektrodenmaterials, beispielsweise durch anodische Oxidation oder Glimmoxidation in an sich bekannter Weise als Zirkonoxid, Titanoxid, Tantaloxid, Wolframoxid oder Molybdänoxid oder Aluminiumoxid auf den Elektroden hergestellt werden. Dabei können Elektrodenüberzüge und Membran im wesentlichen auch aus dem gleichen Grundmaterial, gegebenenfalls mit unterschiedlichen Strukturen, bestehen, so daß die erfindungsgemäßen Eigenschaften von Überzug (flüssigkeitsdicht) und Membran (nicht flüssigkeitsdicht) realisierbar sind.

Bei einer besonders bevorzugten Ausführungsform der erfindungsgemäßen Meßfühler, die sich besonders für die Messung von Ölen eignet, weisen die durchgängigen Poren in der nichtleitenden Membran einen Durchmesser auf, der etwa zwischen 0,01 und 1$\mu$m und bevorzugt zwischen 0,05 und 0,2 $\mu$m liegt.

Ferner kann nach Anspruch 11 auch mindestens eine der Elektroden porös sein und die Innenwände der porösen Elektrode einen nichtleitenden Überzug aufweisen, der im wesentlichen flüssigkeitsdicht ist. Bei dieser Ausführungsform ist es auch möglich, auf die Membran zu verzichten, und beispielsweise die beiden isolierten Elektroden nur "aufeinander" zu legen.

Bei Verwendung einer Membran kann deren Dicke zwischen 10 und 100 $\mu$m und bevorzugt zwischen 20 und 40 $\mu$m betragen.

Bei einer weiteren bevorzugten Ausführungsform des erfindungsgemäßen Meßfühlers, die sich insbesondere für den Einsatz in Wasser, wässrigen Lösungen oder wasserhaltigen Flüssigkeiten eignet, besteht die Membran aus nichtporösem Korund, Aluminiumhydroxylsilkat, z.B. Glimmer oder Gamma-Aluminiumoxid besteht, also Materialien mit Schichtgitter-Aufbau, bei denen Einschlüsse schnell zwischen die einzelnen Gitterschichten eindringen können. Die Membrandicke bei dieser bevorzugten Ausführungsform beträgt zwischen etwa 6 und 100 $\mu$m, bevorzugt 30 bis 40 $\mu$m.

Es kann auch vorgesehen sein, daß der zur Messung in wässrigen Lösungen geeignete Fühler wenigstens teilweise von einer ionenselektiven Membran eingeschlossen ist, welche beispielsweise die Selektivität für Wasserstoff oder Hydroxylionen, Kalium-, Natrium-, Chlorid-, Kalzium- oder Magnesiumionen steigert. Dadurch ist auch eine Bestimmung dieser Ionen möglich.

Die Elektroden können in Art eines Plattenkondensators angeordnet sein, es ist aber auch möglich, daß eine Elektrode die andere Elektrode zylindrisch wie bei einen Zylinderkondensator umgibt.

Bei einer erfindungsgemäßen Vorrichtung zum Messen der Eigenschaft(en) einer Flüssigkeit wird der Meßfühler in die Flüssigkeit eingebracht, eine variable Wechselspannung zwischen 50 Hz und 1 GHz an die Elektroden angelegt und der dielektrische Verlustfaktor D und/oder die Kapazität C in Abhängigkeit von der Meßfrequenz bestimmt. Die derart erhaltenen Meßwerte werden in einer Auswerteeinheit gespeichert, welche aus den entsprechenden Meßwertepaaren dielektrischer Verlustfaktor/Frequenz bzw. der Abhängigkeit der Kapazität von der Frequenz anhand eines Vergleiches mit in der Auswerteeinheit gespeicherten Referenzwerten zur Bestimmung von Eigenschaften der Flüssigkeit, wie der Viskosität, des pH-Wertes, des Ionengehalts und ähnliches befähigt ist.

Wie bereits ausgeführt, besteht eine besonders bevorzugte Einsatzmöglichkeit in der Überwachung von Ölen, od.dgl. wie beispielsweise Motor-, Turbinen-, Pumpen-, Transformatoren und ähnlichen Ölen. Durch den Einsatz erfindungsgemäßer Meßfühler ist es möglich, ständig die Eigenschaften eines im Gebrauch befindlichen Öles zu untersuchen, wobei über die abgespeicherten Meßwerte gegebenenfalls auch der gesteuerte Zusatz von Additiven zu in Gebrauch befindlichem Öl, welche die Eigenschaften des Öls für seinen Gebrauchszweck positiv beeinflußen, möglich ist.

Öle müssen spätestens dann ausgetauscht werden, wenn ihre Funktionstüchtigkeit auf ein gerade noch tragbares Maß abgesunken ist. Bisher wurde das Öl ohne weitere Überprüfung seiner tatsächlichen Alterung nach einem ermittelten mittleren "Wartungszeitraum" ausgetauscht; dies führt bei zu frühem Ölwechsel zu erhöhten Ölkosten, bei zu spätem Ölwechsel andererseits zu einem erhöhten Verschleiß von Motorteilen.

Wichtige Eigenschaften von Motorenschmierölen sind
- die Schmierwirkung (Reibungsverminderung)
- der Korrosionsschutz
- Schutz von Laufteilen gegenüber Einwirkung bzw. Ablagerung von partikelförmigen Verunreinigungen.

Diese Öleigenschaften können dadurch erzielt werden, daß Additive zur Viskositätsverbesserung und für eine ausreichende Schmierwirkung zugesetzt werden. Gegenüber chemischer Korrosion wird das Öl alkalisch eingestellt. Verunreinigungspartikel werden durch Detergentien bzw. Dispergentien in fein verteilter Form im Öl gehalten, so daß es zu keiner Ablagerung oder Ansammlung derartiger Teilchen kommt.

Während des Gebrauchs bzw. Einsatzes des Öls wird durch den oxidativen Abbau

4

(Säuregruppenbildung) die Alkalität vermindert; andererseits vergrößert oder verkleinert sich je nach Betriebsbedingungen die Viskosität. Eine Überprüfung der Funktionstüchtigkeit des Öls, wie sie beispielsweise bei Motoren mit wechselnder Beanspruchung, z.B. bei Motoren in schweren Landfahrzeugen, erwünscht ist, sollte sich daher auf die drei Haupteigenschaften Alkalität, Viskosität und Dispergenz/Detergenz-Additivkonzentration erstrecken.

Es ist bekannt, die qualitativen Eigenschaften von Öl in Labors anhand von Proben, beispielsweise durch Polarographie oder Viskosimetrie festzustellen. Ferner kann der Öldruck ständig gemessen werden, der eine indirekte Aussage über die Viskosität des Öls liefert. Schließlich kann man die Leitfähigkeit des Öls ständig messen, die eine pauschale Aussage über ionische Verunreinigungen im Öl liefert. Diese bekannten Verfahren sind aber für eine kontinuierliche Untersuchung des Öls während des Betriebs des Motors, beispielsweise in einem Kraftfahrzeug während der Fahrt, zu aufwendig, da sie entweder eine Probeentnahme erfordern, also nicht im geschlossenen Motorölkreislauf durchgeführt werden können, oder weil sie verschiedene, sehr große Meßgeräte erfordern.

Durch die erfindungsgemäß geschaffene Meßsonde ist es erstmals möglich, in vorteilhafter Weise die Öleigenschaften laufend zu überwachen. Überraschenderweise ist es möglich, die erfindungsgemäßen Sonden so zu gestalten daß sie trotz kleiner Abmessungen zufriedenstellende Messungen der Viskosität und Alkalität liefern. Dabei geht die Erfindung davon aus, daß die dielektrischen Werte der Öle frequenzabhängig sind. Die poröse, nichtleitende Schicht zwischen den Kondensatorelektroden wirkt sich so aus, daß die dielektrischen Werte bei verschiedenen Frequenzen Maxima oder Stufen zeigen, die typisch für ein bestimmtes Öl bzw. Öleigenschaft sind. Ohne die erfindungsgemäße nichtleitende poröse Schicht zwischen den Elektroden zur Aufnahme des zu messenden Öls ist der frequenzabhängige Verlauf der dielektrischen Werte monoton, es läßt sich somit kein für das Öl spezifisches Maximum erkennen.

Erfindungsgemäß können die Kapazität, die Dielektrizitätszahl und der dielektrische Verlustfaktor gemessen und über einen Rechner ausgewertet werden. Das weiter unten beschriebene Ausführungsbeispiel bezieht sich auf die Verarbeitung des dielektrischen Verlustfaktors; selbstverständlich kann auch aus den anderen dielektrischen Werten wertvolle Information gewonnen werden.

Frequenzabhängige Maxima des dielektrischen Verlustfaktors bei Anlegen von Wechselspannung mit Frequenzen zwischen 50 Hz und 1 GHz sind wahrscheinlich darauf zurückzuführen, daß die ölgefüllten Poren der Membran bei der Betriebstemperatur von etwa 150°C elektrisch leitende Inhomogenitäten in der nichtleitenden Membran darstellen, die zu einem dielektrischen Relaxationsverhalten, welches als Maxwell-Wagner-Effekt bekannt ist, führen. Elektrisch leitende Partikel, die in einem Isolator eingebettet sind, zeigen bei angelegten elektrischen Wechselfeld eine periodische Polarisation. Bei steigender Frequenz wird zunehmend Energie zur Polarisation der Partikel verbraucht. Beim Überschreiten der Relaxationsfrequenz ($f_R$) wird die Amplitude der Ladungsträger kleiner. Man erhält daher ein Maximum des dielektrischen Verlustes bei der Relaxationsfrequenz und eine Stufe in der Dielektrizitätszahl. Das Öl stellt somit schlecht elektrisch leitende Inhomogenitäten im nichtleitenden Membranmaterial dar und zeigt deshalb den Maxwell-Wagner-Effekt.

Dabei hängt die Konzentration der Ladungsträger im Öl vom Gehalt an sauren, alkalischen und salzartigen Gruppen oder Degradationsprodukten des Öls sowie dem Wassergehalt ab. Bei Erhöhung der Frequenz nehmen zunächst alle diese als Ladungsträger wirkenden Gruppen an der aufgezwungenen periodischen Bewegung teil, fallen aber mit steigender Frequenz aufgrund ihrer inhärenten Trägheit nach und nach aus. Dies geschieht bei einer für jede Ladungsträgersorte charakteristischen Relaxationsfrequenz mit einem mehr oder weniger ausgeprägten Maximum im dielektrischen Verlustfaktor D oder einer Stufe in der Dielektrizitätszahl oder der Kapazität.

Bei freiem Öl, das nicht in einer Membran eingeschlossen ist, liegen diese Relaxationsfrequenzen unterhalb von 50 Hz und lassen sich schlecht auflösen. Ferner sind die Verluste in freiem Öl zu groß, als daß ein hinreichend großer Meßeffekt beobachtet werden könnte. Die erfindungsgemäßen Membranen führen durch ihre Poren dazu, daß sich eine Raumladungsschicht aufgrund physikalischer Adsorption von Ölbestandteilen am Membranmaterial ausbildet. Aufgrund dieser Raumladungsschicht erhöht sich die elektrische Leitfähigkeit des Öls beträchtlich, beispielsweise um den Faktor 10; damit verschieben sich auch die zur Leitfähigkeit proportionalen Relaxationsfrequenzen zu höheren Werten, d.h.in einen meßtechnisch günstigeren Frequenzbereich.

Die erfindungsgemäße Meßsonde kann nun entweder im Durchströmverfahren (insbesondere als Zylinderkondensator) oder im Eintauchverfahren angewendet werden.

Bei Anwendung des erfindungsgemäßen Meßfühlers zur pH-Messung und/oder zur Messung der Ionenaktivität in einer wasseraufweisenden Flüssigkeit kann es ferner vorteilhaft sein, die Membran zur Aufnahme von Wasserstoff-, Hydroxyl- und/oder anderen Ionen befähigt, auszugestalten.

Dadurch, daß Hydroxyl- und/oder Wasserstoffionen oder andere Ionen in die Membran eindringen, ändert sich das dielektrische Verhalten des Kondensators.

Der erfindungsgemäße pH-Fühler, der im wesentlichen nur auf $OH^-$ und $H^+$ reagiert, ist besonders vorteilhaft, da er - im Gegensatz zu den bisher üblichen Glas-pH-Meßsonden - nicht leicht zerbrechlich ist und außerdem auch bei extremen pH-Werten, insbesondere im Alkalischen, arbeiten kann. Glaselektroden sind in stark alkalischen Lösungen nicht einsetzbar. Ein weiterer Nachteil der bekannten pH-Meßfühler besteht außer in ihrer Empfindlichkeit auch darin, daß sie relativ voluminös sind und somit für kleinste Meßvolumina nicht einsetzbar sind.

Durch die erfindungsgemäße Ausbildung, insbesondere dadurch, daß eine widerstandsfähige Membran zwischen überzogene Elektroden eingesetzt wird, ist es möglich, beispielsweise den pH-Wert oder andere Ionenaktivitäten in galvanischen Bädern, Elektrolytbädern, Bioreaktoren und ähnlichen zu überwachen. Dabei ist insbesondere eine kontinuierliche Messung, sogar bei extrem alkalischen pH-Werten, möglich.

Der Meßfühler gemäß der Erfindung kann auch für die Messung von anderen Ionen, beispielsweise Kalium-, Natrium-, Chlor-, Kalzium- oder Magnesiumionen eingerichtet werden, indem eine 15 bis 20 $\mu$m-dicke Kapsel einer ionenselektiven Membran um Elektroden, Membran und Elektrodenüberzüge gebildet wird, welche die zu messenden Ionen selektiv durchläßt. Voraussetzung für die Messung derartiger Ionen ist, daß diese in das Membranmaterial eindringen können.

## Kurze Beschreibung der Zeichnung

Im folgenden soll die Erfindung anhand von Ausführungsbeispielen unter Bezugnahme auf die Zeichnung näher beschrieben werden, in der zeigen:

Fig. 1    einen Querschnitt durch einen erfindungsgemäßen Meßfühler zur Erläuterung des prinzipiellen Aufbaus,

Fig. 2    eine Ansicht einer möglichen Ausführungsform eines erfindungsgemäßen Meßfühlers,

Fig. 3    eine mit einem erfindungsgemäßen Meßfühler erhaltene Meßkurve D(f) für Neu- und Altöl,

Fig. 4    eine Meßkurve für die Bestimmung der Alkalität,

Fig. 5    die Verschiebung von Dielektrizitätsverlust-Maxima in Abhängigkeit von der Frequenz bei verschiedenen  Materialien;

Fig. 6    die Abhängigkeit von $K_\eta$ von $\eta$;

Fig. 7    die Änderung von D bei Additivzugabe zu Motorenöl.

## Wege zur Ausführung der Erfindung

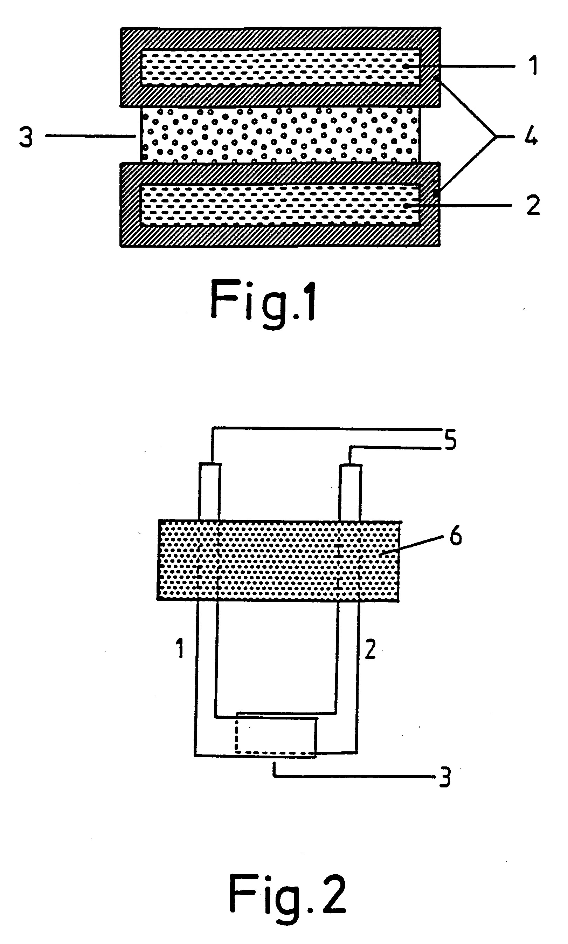

Fig. 1 zeigt nichtmaßstäblich und schematisch den Aufbau eines als Plattenkondensator ausgebildeten erfindungsgemäßen Meßfühlers. Der Meßfühler weist zwei Elektroden 1 und 2 auf, auf denen sich ein elektrisch isolierender Überzug 4 befindet und zwischen denen eine Membran 3 angeordnet ist.

Mit den beiden Elektroden 1 und 2 des Meßfühlers ist eine Signalverarbeitungs- und Auswerteeinheit verbunden, die eine Wechselspannung mit variabler Frequenz an die Elektroden anlegt. Aus dem komplexen Widerstandsverhalten des Meßfühlers ermittelt die Auswerteeinheit die Kapazität und/oder den dielektrischen Verlustfaktor des Kondensators. Diese Größen können wie im folgenden gezeigt werden wird aufgrund des Aufbaus des erfindungsgemäßen Meßfühlers mit hoher Genauigkeit und Reproduzierbarkeit zur Bestimmung der zu messenden Flüssigkeitseigenschaften herangezogen werden.

Da der Aufbau einer Signalverarbeitungs- und Auswerteinheit, die die Kapazität und/oder den dielektrischen Verlustfaktor eines Kondensators ermittelt, an sich bekannt ist, muß er an dieser Stelle nicht weiter erläutert werden. Die Auswertung kann dabei analog oder digital, z.B. mit einen Mikrocomputer oder einem Universalrechner erfolgen.

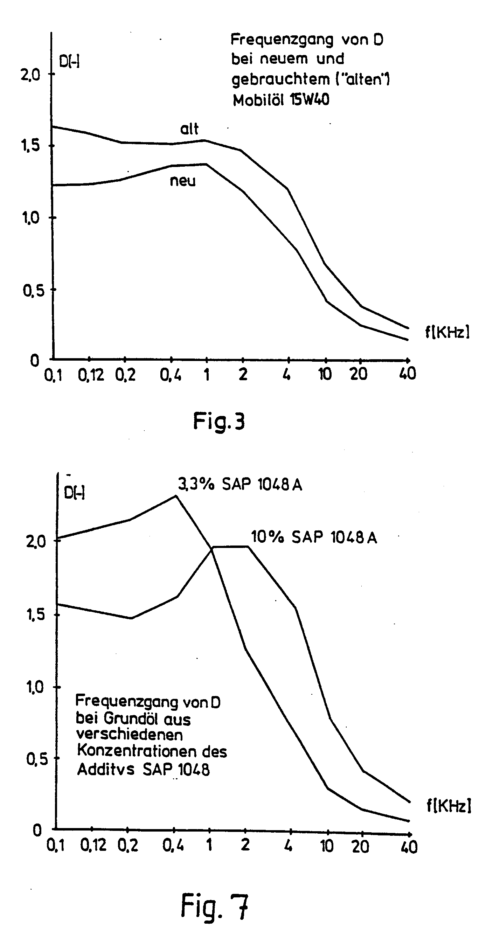

Fig. 1 zeigt lediglich den prinzipiellen Aufbau. Eine in der Praxis realisierte Ausführungsform ist in Fig.2 dargestellt. Bei dieser Ausführungsform sind die beiden Elektroden winkelförmig ausgebildet; die beiden winkelförmigen Teile schließen zwischen sich die Membran 3 ein. Eine Elektrodenhalterung 6 fixiert die Lage der Elektroden 1 und 2 gegeneinander; am Ende der Elektroden sind Zuleitungen 5 für das Anlegen der Wechselspannung bzw. den Anschluß der Auswerteeinheit vorgesehen.

Im folgenden soll zunächst ein Ausführungsbeispiel für einen erfindungsgemäßen Meßfühler zur Messung von wasserhaltigen Lösungen und insbesondere des pH-Werts von wasserhaltigen Lösungen und anschließend ein Ausführungsbeispiel beschrieben werden, das sich vor allem zur Messung in technischen Ölen und insbesondere zur Messung deren Alkalität, Viskosität und Dispergenz/Detergenz-Additivkonzentration eignet.

pH-Fühler

Bei dem im folgenden dargestellten Ausführungsbeispiel sind die beiden Elektroden 1 und 2 aus Titan hergestellt und weisen einen Rutil oder Titanaluminiumzirkonatüberzug 4 auf. Der Überzug 4 ist nicht porös und etwa 8 bis 30 $\mu$m dick. Die Membran 3 zwischen den Elektroden ist etwa 10 bis 100 $\mu$m dick und besteht aus nichtporösem Korund oder Gamma-Aluminiumoxid. Der erfindungsgemäße Meßfühler kann bei Verwendung als pH-Fühler folgende Abmessungen aufweisen: Dicke 0,5 mm, Breite 5 mm und Länge: 10 mm.

Die Frequenz der von der Signalverarbeitungs und Auswerteeinheit angelegten Wechselspannung variiert im Bereich zwischen etwa 50 Hz und 1 GHz und bevorzugt zwischen 100 Hz und 40 KHz, der Spitzenwert der Spannung liegt bevorzugt in der Größenordnung 1V.

Setzt man einen derartigen Meßfühler in die zu messende Flüssigkeit, beispielsweise eine wässrige Lösung ein, so dringen Ionen in die Membran ein. Die dielektrischen Eigenschaften der Membran 3 ändern sich insbesondere durch das Eindringen von Wasserstoff und Hydroxylionen, so daß Kapazität und dielektrischer Verlustfaktor mit dem pH-Wert korreliert sind. In den nichtporösen Überzug 4 der Elektroden 1 und 2 andererseits diffundieren keine Ionen ein, so daß die Elektroden 1 und 2 weiter isoliert sind, und der komplexe Widerstand des Meßfühler Kondensators nicht durch eine galvanische Überbrückung, sondern lediglich durch die Änderung der dielektrischen Eigenschaften der Membran 3 verändert wird. Da die Membran 3 chemisch durch die Flüssigkeit nicht angegriffen wird, die lediglich in Poren bzw. in das Material (z.B. bei Glimmer) selbst eindringt, ist die Reproduzierbarkeit hoch.

Die Auswerteinheit speichert die Meßwerte in Zuordnung zur jeweiligen Meßfrequenz und vergleicht die Meßwerte mit eingespeicherten Eichdaten. Aus dem Vergleich der Meßdaten mit den gespeicherten Eichdaten können die pH-Werte mit großer Genauigkeit und Reproduzierbarkeit ermittelt werden. Durch die große Reproduzierbarkeit ist es möglich, die Auswerteeinheit so "vorzueichen", daß sie direkt den pH-Wert als Meßergebnis ausgibt.

Bei einer besonders vorteilhaften Weiterbildung des pH-Fühlers, die sich insbesondere für Medizin und Biotechnik eignet, wird eine 5 bis 20 $\mu$m dicke ionenselektive Membrankapsel um den erfindungsgemäßen Fühler gelegt, welche beispielsweise die Selektivität für Wasserstoff- und Hydroxylionen bzw. Kalium-, Natrium-, Chlor- oder Kalciumionen steigert. Durch diese selektive Störung ist es möglich, diese Ionen getrennt bzw. separat von anderen Stör-Ladungen in der Flüssigkeit zu ermitteln.

Öl-Fühler

Die qualitativen Eigenschaften von Motorölen und deren Gemischen, welche im folgenden kurz als Öl bezeichnet werden, ändern sich durch Alterung bei Belastung des Öls. Es ist daher notwendig, die Öle nach einer bestimmten Zeit auszutauschen. Da der "Verbrauch" des Öls aber nicht ständig überprüft wird, wird häufig das Öl zu früh gewechselt, oder verbleibt in anderen Fällen zu lange in seinem Einsatzgebiet, beispielsweise einer Turbine, einem Motor, einer Vakuumpumpe oder einem Transformator.

Zunächst soll ein Ausführungsbeispiel beschrieben werden, bei dem die Elektroden 1 und 2 wie bei den in Fig. 1 und 2 gezeigten Ausführungsformen plattenförmig ausgebildet sind. Die Elektroden bestehen beispielsweise aus Reintitan oder aus einer Titanlegierung, die bis zu 6% Aluminium, bis zu 4% Vanadium und evtl. Spuren von Kupfer enthält.

Die Elektroden 1 und 2 sind wiederum mit einem nichtleitenden Überzug 4 versehen, der durch anodische oder Glimm-Oxidation des Elektrodenmetalls hergestellt werden kann. Bei dem erläuterten Ausführungsführungsbeispiel ist auf den Elektroden aus Titan eine Titan(di)oxidschicht mit einer Dicke zwischen etwa 8 $\mu$m und 20 $\mu$m durch anodische Oxidation in wässriger Natriumsulfat-Lösung gebildet. Ausdrücklich wird darauf hingewiesen, daß die Eigenschaften der Titan(di)oxidschicht von der Stromdichte und der Spannung bei der Herstellung abhängen; die Verfahrensparameter bei der Herstellung des Überzugs 4 auf den Elektroden sind deshalb entsprechend den gewünschten Eigenschaften des Überzugs einzustellen.

Die nichtleitende poröse Membran 3 hat vorzugsweise eine Dicke zwischen etwa 10 $\mu$m und 100 $\mu$m; der Porendurchmesser sollte zwischen 0,01 $\mu$m und 1,0 $\mu$m, bevorzugt zwischen 0,05 und 0,2 $\mu$m betragen.

Eine derartige Membran kann beispielsweise dadurch hergestellt werden, daß eine Aluminiumfolie mit einer Dicke von 10 $\mu$m bis 100 $\mu$m in 15 %iger wässriger $H_2SO_4$-Lösung bei konstanten Stromdichten von ca. 10 mA/cm$^2$ vollständig durchoxidiert wird. Zweckmäßigerweise hat die Schicht Poren mit einer spezifischen Oberfläche in der Größenordnung 100 m$^2$/g.

Ein Fühler mit dem vorstehend genannten Aufbau hat z.B. folgende typische Abmessungen: Breite: 2mm, Länge: 25 mm, Dicke: 0,2 mm

Die Elektroden können rechteckig sein, so daß sie einen "Plattenkondensator" bilden, es können aber auch zylinderförmige Elektroden verwendet werden.

Typische Dimensionen der zylinderförmigen Elektroden sind:

Durchmesser der inneren Elektrode 1 bis 10 mm; diese Elektrode kann massiv oder ein Hohlzylinder mit einer Wandstärke von bspw. 0,5 bis 3 mm sein. Die äußere Elektrode ist ein Hohlzylinder mit einem Innendurchmesser, der gleich dem Außendurchmesser der Innenelektrode zuzüglich der Dicke der porösen Schicht ist; ihr Außendurchmesser liegt zwischen 2 und 15 mm. Die Länge des aktiven Teils der zylinderförmigen Elektrode beträgt etwa 1 bis 5 mm.

Um einen leichteren Öldurchtritt zur porösen Zwischenschicht zu ermöglichen, kann mit Vorteil mindestens eine der beiden Elektroden derart porös ausgebildet werden, daß auch die Innenwände der Poren mit Metalloxid überzogen sind.

An die Elektroden wird durch eine elektronische Auswerteeinheit eine Wechselspannung angelegt, deren Frequenz zwischen etwa 0,05 kHz und 1 GHz variierbar ist und deren Spannungsspitzenwert zwischen etwa 1V und 5V liegt. Aus dem Verhalten, welches die Meßsonde als frequenzabhängiger komplexer Widerstand zeigt, wird durch die Auswerteeinheit eine charakteristische dielektrische Größe des Meßfühlers im Ausführungsbeispiel der dielektrische Verlustfaktor D, als Funktion der Meßfrequenz f, $D = D(f)$, ermittelt. Im frequenzabhängigen Verlauf der dielektrischen Größe treten infolge der bereits erwähnten eigentümlichen Wirkung der porösen nichtleitenden Membran 3 des Meßfühlers markante Werte, im Beispiel Maxima von D, auf, von denen die Figuren 3, 5 und 7 zwischen 0,1 und 10 kHz gelegene Maxima zeigen. Ein diesen vorausgehendes Maximum tritt unter 0,1 kHz auf, es ist in den Figuren nicht erfaßt. Weitere, in den Figuren ebenfalls nicht dargestellte Maxima ergeben sich bei 40 kHz überschreitenden Werten der Meßfrequenz f. Zu den Maxima von D gehören die Relaxationsfrequenzen $f_R$ als jeweilige markante Werte der Meßfrequenz f.

In Fig. 3 ist die Abhängigkeit des dielektrischen Verlustfaktors als Funktion der angelegten Frequenz bei neuem und gebrauchtem ("altem") Motorenöl des Typs 15W40 dargestellt. Es zeigt sich sogleich, daß sich der Kurvenverlauf bei Altöl zu größeren dielektrischen Verlustfaktoren bei gleichen Meßfrequenzen verschiebt; bei diesem Öl tritt bei $f_R$ von 1 kHz ein Maximum von D auf, welches von D = 1,40 auf etwa D = 1,55 steigt.

Aus der Figur geht hervor, daß sich im Zuge der Alterung auch die mittlere Steigung der zwischen der Ordinatenachse und dem jeweiligen Maximum von D gelegenen Kurvenabschnitte geändert hat, und zwar ist sie tendenziell kleiner geworden. Dies wird darauf zurückgeführt, daß das bereits erwähnte, unterhalb von 0,1 kHz auftretende, in der Figur nicht gezeigte Maximum überwiegend auf die Azidität des Öls zurückzuführen ist, und diese ist im Laufe der Alterung relativ größer geworden als die Alkalität, die sich überwiegend in den gezeigten Maxima bei etwa 1 kHz äußert.

Die alterungsabhängige Änderung der mittleren Steigung von $D = D(f)$ vor Erreichen der Relaxationsfrequenz $f_R$ wird erfindungsgemäß zur Ermittlung der Alkalität ausgewertet und zwar wird durch die Auswerteeinheit für mindestens zwei Frequenzen, $f_1$ und $f_2$ das Verhältnis

$$A = (D(f_1) - D(f_2)) / (f_2 - f_1)$$

bei einem Funktionsverlauf gemäß der Figur 3 zum Beispiel A = (D(400 Hz) - D(100 Hz))/300Hz berechnet und jeweils als Maß für die augenblickliche Alkalität angezeigt. Der auf diese Weise ermittelte Wert des Verhältnisses A kann ferner erfindungsgemäß auch durch die Auswerteeinheit gespeichert und zur Anzeige der Änderung der Alkalität mit früheren Werten von A verglichen werden.

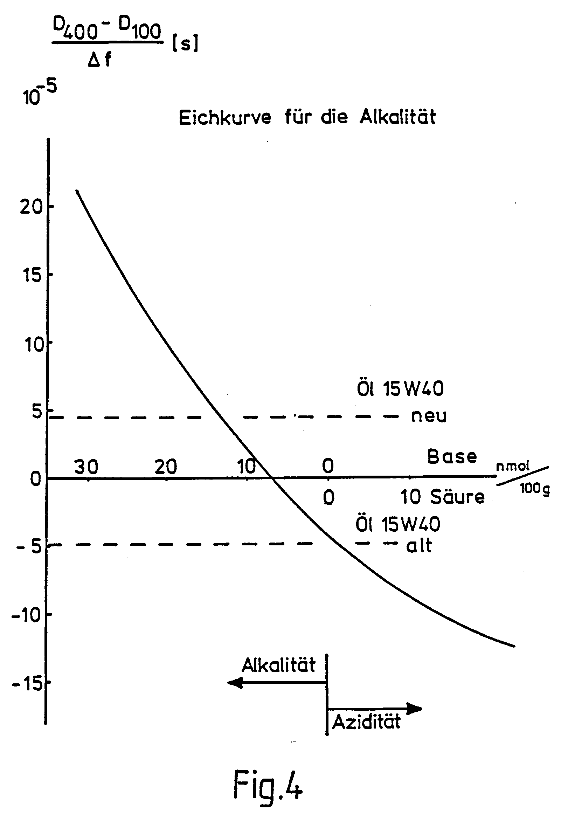

Fig. 4 zeigt die Entwicklung von

$$A = (D(400 Hz) - D(100 Hz))/\Delta f$$

Der Wert der Alkalität A nimmt mit fallendem pH-Wert steil ab; aus dieser Beziehung läßt sich somit zuvrlässig das Auftreten saurer Gruppen in alkalischem Öl bestimmen.

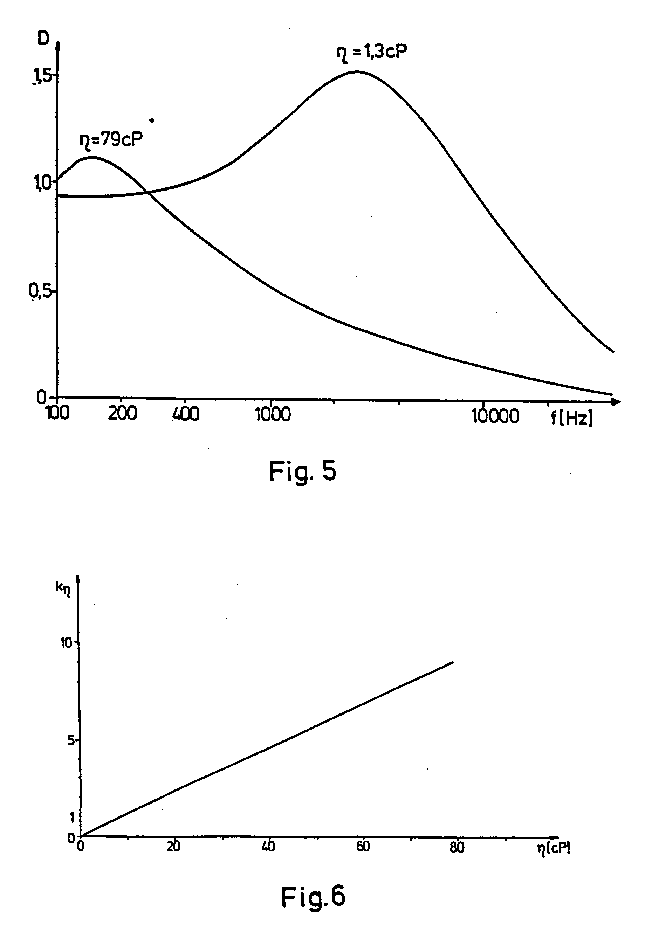

In Fig. 5 sind Meßwerte von $D = D(f)$ mit Ölen der Viskositäten $\eta = 1,3$ cP und $\eta = 79$ cP dargestellt. Mit steigenden Werten von $\eta$ verschieben sich die Relaxationsfrequenzen der D-Maxima von etwa 1,3 kHz zu etwa 150 Hz. Dieser als Maximumverschiebung bezeichnete Erscheinung kann zur Bestimmung der Viskosität $\eta$ eines Öls verwendet werden.

Allgemein ist die relative Zunahme der Maximum-Frequenzen $\Delta f/f$ ein Maß für die relative Abnahme der Viskosität $\Delta\eta/\eta$.

In an sich bekannter und üblicher Weise kann diese Beziehung mit einem Eichfaktor versehen werden und zur Auswertung vom Öleigenschaften, bevorzugt mit einem Rechner, eingesetzt werden.

In Fig. 6 ist der Zusammenhang zwischen Viskosität und Relaxationszeit dargestellt, wobei die dort

EP 0 182 795 B1

dargestellte Größe $k_n = \tau * 10^3$ und $= \Pi * f/2$ ist.

Die Kurve wurde bei gleichbleibender Alkalität aufgenommen.

Die Fig. 5 und 6 beziehen sich auf eines der im Verlauf von D = D(f) auftretenden Maxima, wobei die steigende Gerade einem bestimmten D-Maximum zuordenbar ist. Für mehrere Maxima ergibt sich ein Büschel entsprechender Geraden mit Beginn vom Koordinatenursprung mit unterschiedlichen Steigungen. Da die Lage der Maxima auch von der Leitfähigkeit und damit von der Alkalität des Öls beeinflußt wird, ist es vorteilhaft, diesen Einfluß dadurch zu eliminieren, daß mehrere Maxima berücksichtigt und die Maximumverschiebungen mit mehr als 2 bis 4 Frequenzen (f) erfaßt werden.

Bei gleichzeitig starker Änderung der Alkalität empfiehlt es sich, nach dem Schema

$$\Delta \tau / \tau \;=\; -\,\frac{\Delta f}{f}\times k_1 \;+\; \frac{\Delta b}{b}\times k_2$$

auch die relative Alkalitätsänderung $\Delta b : b$ zu berücksichtigen, wobei die k als Eichfaktoren eingeeicht werden.

Zur Ermittlung der Änderung der Konzentration der Additive des Öls wird erfindungsgemäß die Abhängigkeit der Konzentration vom Wert eines bestimmten Maximums von D, bevorzugt durch Zugabe der betreffenden Aditive zum Öl, aufgenommen, und in die Auswerteeinheit eingespeichert. Die Änderung des durch die Auswerteeinheit festzustellenden augenblicklichen Wertes dieses Maximums gegenüber seinem Ausgangswert kann als Maß für die Änderung der Konzentration angezeigt werden.

In Fig. 7 ist eine Verschiebung des Maximums von D = 2,0 zu D = 2,3 bei einer Abnahme der Konzentration des Additivs von 10 % auf 3,3 % dargestellt.

Vorstehend ist die Erfindung exemplarisch beschrieben worden. Im Rahmen des allgemeinen Erfindungsgedankens sind die verschiedensten Modifikationen möglich: Beispielsweise ist es möglich, mehrere Fühler zuverwenden, um die einzelnen Öleigenschaften getrennt zu erfassen.

**Patentansprüche**

1. Meßfühler zur Untersuchung von Flüssigkeiten,
   mit zwei Elektroden (1,2), die einen elektrisch isolierenden und im wesentlichen flüssigkeitsdichten Überzug (4) aufweisen, und
   mit einer zwischen den Elektroden (1,2) angeordneten nichtleitenden Membran (3), die aus einem flüssigkeitsdichten Metalloxid, Metallmischoxid oder einem anorganischen oder organischem Isolationsmaterial besteht, das durchgängige Poren, Spalte oder Zwischenräume mit einer großen Oberfläche, beispielsweise in der Größenordnung von $100 m^2/g$ aufweist in die die zu untersuchende Flüssigkeit eindringen kann, so daß sich durch physikalische Adsorption eine Raumladungsschicht ergibt.

2. Meßfühler nach Anspruch 1,
   dadurch **gekennzeichnet,** daß die Elektroden (1,2) im wesentlichen aus Aluminuim, Zirkon, Titan, Tantal, Wolfram und/oder Molybdän oder einem ähnlichen Metall bzw. Legierungen dieser Metalle bestehen.

3. Meßfühler nach Anspruch 1 oder 2,
   dadurch **gekennzeichnet**, daß die nichtleitende Membran (3) im wesentlichen aus Aluminiumoxid, Siliziumnitrid, Titanoxid, $[KAl_2(OH)_2] * [AlSi_3O_{10}]$ oder Polytetrafluorethylen besteht.

4. Meßfühler nach einem der Ansprüche 1 bis 3,
   dadurch **gekennzeichnet,** daß die elektrisch isolierenden Überzüge (4) der Elektroden (1,2) im wesentlichen aus einem nichtleitenden Metalloxid, Metallmischoxid, Metallnitrid oder einem anderen anorganischen Isolationsmaterial bestehen.

5. Meßfühler nach einem der Ansprüche 1 bis 4,
   dadurch **gekennzeichnet,** daß die Überzüge (4) aus amphoteren Oxiden, wie $Al_2O_3$ oder Rutil, oder aus Mischoxiden, wie Titanaluminiumzirkonat, bestehen.

9

**6.** Meßfühler nach einem der Ansprüche 1 bis 5,
dadurch **gekennzeichnet,** daß die Überzüge durch direkte Oxidation des Elektrodenmaterials zu Zirkonoxid, Titanoxid, Tantaloxid, Wolframoxid, Molybdänoxid, Aluminiumoxid oder einem ähnlichen Übergangsmetalloxid, beispielsweise durch anodische oder Glimmoxidation, hergestellt sind.

**7.** Meßfühler nach einem der Ansprüche 1 bis 6,
dadurch **gekennzeichnet,** daß die Dicke des Überzugs zwischen etwa 5 $\mu$m und 30 $\mu$m beträgt.

**8.** Meßfühler nach einem der Ansprüche 1 bis 7,
dadurch **gekennzeichnet,** daß die Elektroden (1,2) aus Reintitan oder einer Titanlegierung, beispielsweise mit einem Gehalt von bis zu 6% Aluminium und bis zu 4% Vanadin und gegebenenfalls Kupfer, die Elektrodenüberzüge (4) aus $TiO_2$ und die Membran (3) aus $Al_2O_3$ bestehen.

**9.** Meßfühler nach einem der Ansprüche 1 bis 7,
dadurch **gekennzeichnet,** daß die Überzüge (4) und Membran (3) im wesentlichen aus dem gleichen chemischen Grundmaterial, gegebenenfalls mit unterschiedlichen Strukturen, bestehen.

**10.** Meßfühler nach einem der Ansprüche 1 bis 9,
dadurch **gekennzeichnet**, daß der Durchmesser der Poren der Membran (3) zwischen etwa 0,01 $\mu$m und 1$\mu$m, bevorzugt zwischen 0,05 $\mu$m und 0,2 $\mu$m beträgt.

**11.** Meßfühler nach einem der Ansprüche 1 bis 10,
dadurch **gekennzeichnet,** daß mindestens eine Elektrode (1, 2) porös ist, und die porösen Innenwände der Elektrode einen nichtleitenden flüssigkeitsdichten Überzug (4) aufweisen.

**12.** Meßfühler nach einem der Ansprüche 1 bis 11,
dadurch **gekennzeichnet,** daß die Membrandicke 10 $\mu$m bis 100 $\mu$m, bevorzugt 20 $\mu$m bis 40 $\mu$m beträgt.

**13.** Meßfühler nach einem der Ansprüche 1 bis 12,
dadurch **gekennzeichnet,** daß die nichtporöse Membran (3) aus einem Material mit Schichtaufbau besteht, in das die zu untersuchende Flüssigkeit eindringen kann.

**14.** Meßfühler nach Anspruch 13,
dadurch **gekennzeichnet,** daß die Membran aus nichtporösem Korund, Aluminium-Hydroxylsilikat oder $\gamma$-Aluminiumoxid besteht.

**15.** Meßfühler nach Anspruch 14,
dadurch **gekennzeichnet,** daß die Membrandicke zwischen etwa 6 und 100 $\mu$m, bevorzugt 30 bis 40 $\mu$m, beträgt.

**16.** Meßfühler nach einem der Ansprüche 1 bis 15,
dadurch **gekennzeichnet,** daß der Fühler mindestens teilweise von einer ionenselektiven Membran eingeschlossen ist.

**17.** Meßfühler nach Anspruch 16,
dadurch **gekennzeichnet,** daß die ionenselektive Membran die Selektivität für Wasserstoff oder Hydroxylionen, Kalium-, Natriumchlorid-, Magnesium- oder Kalzium-Ionen steigert.

**18.** Meßfühler nach einem der Ansprüche 1 bis 17,
dadurch **gekennzeichnet,** daß die Elektroden (1,2) einander gegenüberliegend in Art eines Plattenkondensators angeordnet sind.

**19.** Meßfühler nach einem der Ansprüche 1 bis 17,
dadurch gekennzeichnet, daß eine Elektrode (1) die andere Elektrode (2) zylindrisch umgibt, wobei die Membran die innere Elektrode umschließt.

**20.** Vorrichtung zum Messen von Eigenschaften einer Flüssigkeit mit einem Meßfühler nach einem der

EP 0 182 795 B1

Ansprüche 1 bis 19, der in die zu untersuchende Flüssigkeit eingetaucht ist,
dadurch **gekennzeichnet,** daß eine Signalverarbeitungs- und Auswerteeinheit an die beiden Elektroden (1,2) eine Wechselspannung anlegt, die Frequenz der Wechselspannung zwischen 50 Hz und 1 GHz variiert, und den dielektrischen Verlustfaktors D = D(f) und/oder der Kapazität C = C(f) bei verschiedenen Frequenzen mißt, und
daß die Auswerteeinheit die gemessenen Werte D(f) und/oder C(f) mit gespeicherten Referenzwerten $D_{ref}$(f) bzw. $C_{ref}$(f) vergleicht.

21. Vorrichtung nach Anspruch 20,
dadurch **gekennzeichnet,** daß die Auswerteinheit zur Bestimmung der Alkalität insbesondere eines Öls den Wert A entsprechend der Beziehung

$$A = ( D(f_2) - D(f_1) ) / (f_2 - f_1)$$

berechnet, wobei
$D(f_i)$ der dielektrische Verlustfaktor bei der Frequenz $f_i$ ist, und
den gemessenen Wert A mit einem gespeicherten Referenzwert $A_{ref}$ vergleicht, und die Abweichung oder den gemessenen und gegebenenfalls korrigierten Wert A direkt als Maß für die Alkalität ausgibt.

22. Vorrichtung nach Anspruch 20 oder 21,
dadurch **gekennzeichnet,** daß die Auswerteeinheit zur Bestimmung der Viskosität einer Flüssigkeit ein oder mehrere Maxima des dielektrischen Verlustfaktors D(f) als Funktion der Frequenz der angelegten Wechselspannung ermittelt und aus dem Vergleich der Frequenzen mit Referenzfrequenzen, bei denen Maxima in einer Referenzflüssigkeit auftreten, die Viskosität der untersuchten Flüssigkeit bestimmt.

23. Vorrichtung nach einem der Ansprüche 20 bis 22,
dadurch **gekennzeichnet,** daß die Auswerteeinheit aus der Höhe der Maxima die Additivkonzentration bestimmt.

24. Vorrichtung nach einem der Ansprüche 20 bis 23,
dadurch **gekennzeichnet,** daß die Auswerteeinheit die gemessenen Kapazitäts-/Frequenzwertepaare mit abgespeicherten Kapazitäts-/Frequenzwertepaaren vergleicht und den Wertepaaren einen pH-Wert zuordnet.

25. Vorrichtung nach einem der Ansprüche 20 bis 24,
dadurch **gekennzeichnet,** daß die Auswerteeinheit die gemessenen Dielektrizitäts-Verlustfaktor/Frequenzwertpaare mit abgespeicherten Dielektrizitäts-Verlustfaktor/Frequenzwertpaaren vergleicht.

26. Vorrichtung nach einem der Ansprüche 20 bis 25,
dadurch **gekennzeichnet,** daß die Auswerteeinheit bei Überschreiten eines vorgegebenen Grenzwertes von $D(f_r)$ und/oder $C(f_r)$ oder von aus diesen Größen berechneten Daten eine Anzeige und/oder Steuersignal für eine Stellgröße, beispielsweise eine Ventilsteuerung eine Pumpe oder ähnliches abgibt.

**Claims**

1. Measuring probe for the analysis of liquids, comprising two electrodes (1, 2) provided with an electrically insulating and essentially fluid-tight coating (4), and
a non-conducting diaphragm (3) which is disposed between said electrodes (1, 2), which consists or a liquid-tight metal oxide, a mixed metal oxide, or an inorganic or organic insulating material including continuous pores, gaps or spaces having a large surface, e.g. in the range or 100 $m^2$/g, for the penetration of the liquid to be analysed therein, so as to achieve a volume charge layer as a result of physical adsorption.

2. Measuring probe according to Claim 1,
**characterized** in that said electrodes (1, 2) substantially consist of aluminium, zirconium, titanium, tantalum, tungsten and/or molybdenum or a similar metal or alloys of such metals.

3. Measuring probe according to Claim 1 or 2,
   **characterized** in that said non-conducting diaphragm (3) substantially consists or alumina, silicon nitride, titanium oxide, [KAl$_2$(OH)$_2$] * [AlSi$_3$O$_{10}$] or polytetrafluoroethylene.

4. Measuring probe according to any of Claims 1 to 3,
   **characterized** in that said electrically insulating coatings (4) on said electrodes (1, 2) substantially consist of a non-conducting metal oxide, mixed metal oxide, metal nitride, or any other inorganic insulating material.

5. Measuring probe according to any of Claims 1 to 4,
   **characterized** in that said coatings (4) consist or amphoteric oxides such as Al$_2$O$_3$ or rutile, or mixed oxides such as titanium-aluminium zirconate.

6. Measuring probe according to any of Claims 1 to 5,
   **characterized** in that said coatings are produced by direct oxidation or the electrode material, e.g. by anodic or glow oxidation, so as to form zirconium oxide, titanium oxide, tantalum oxide, tungsten oxide, molybdenum oxide, aluminium oxide or a similar transition metal oxide.

7. Measuring probe according to any or Claims 1 to 6,
   **characterized** in that the thickness of the coating is in the range from 5 $\mu$m to 30 $\mu$m.

8. Measuring probe according to any or Claims 1 to 7,
   **characterized** in that said electrodes (1, 2) consist of pure titanium or a titanium alloy, e.g. containing up to 6% aluminium and up to 4% vanadium and possibly copper, while said electrode coatings (4) consist of TiO$_2$ and said diaphragm (3) is made from Al$_2$O$_3$.

9. Measuring probe according to any of Claims 1 to 7,
   **characterized** in that said coatings (4) and said diaphragm (3) consist essentially of the same chemical basic material, if necessary of different structures.

10. Measuring probe according to any of Claims 1 to 9,
    **characterized** in that the diameter of the pores in said diaphragm (3) is in the range from roughly 0.01 $\mu$m to 1 $\mu$m, preferably between 0.05 $\mu$m and 0.2 $\mu$m.

11. Measuring probe according to any of Claims 1 to 10,
    **characterized** in that at least one of said electrodes (1, 2) is porous and that the porous inner walls of the electrode are provided with a non-conducting liquid-tight coating (4).

12. Measuring probe according to any of Claims 1 to 11,
    **characterized** in that the diaphragm thickness is 10 $\mu$m to 100 $\mu$m, preferably 20 $\mu$m to 40 $\mu$m.

13. Measuring probe according, to any of Claims 1 to 12,
    **characterized** in that said non-porous diaphragm (3) consists of a material having a stratified structure which may be penetrated by the liquid to be analysed.

14. Measuring probe according to Claim 13,
    **characterized** in that said diaphragm consists of non-porous corundum, aluminium hydroxyl silicate or gamma-alumina.

15. Measuring probe according to Claim 14,
    **characterized** in that the diaphragm thickness is in the range from roughly 6 to 100 $\mu$m, preferably 30 to 40 $\mu$m.

16. Measuring probe according to any of Claims 1 to 15,
    **characterized** in that said probe is enclosed, at least in part, by an ion-selective diaphragm.

17. Measuring probe according to Claim 16,
    **characterized** in that said ion-selective diaphragm enhances the selectivity for hydrogen or hydroxyl ions, as well as ions of potassium, sodium chloride, magnesium or calcium.

18. Measuring probe according to any of Claims 1 to 17,
**characterized** in that said electrodes (1, 2) are disposed opposite each other in the manner of a plate capacitor.

19. Measuring probe according to any of Claims 1 to 17,
**characterized** in that one electrode (1) cylindrically surrounds the other electrode (2), with said diaphragm enclosing the inner electrode.

20. Apparatus for measuring the properties of a liquid, using a measuring probe according to any of Claims 1 to 19, which is immersed into the liquid to be analysed,
**characterized** in that a signal processing and evaluating unit applies an a.c. voltage to said two electrodes (1,2), with the frequency of said a.c. voltage varying between 50 Hz and 1 GHz, and measures the dielectric loss factor D = D(f) and/or the capacitance C = C(f) for different frequencies, and
that said evaluating unit compares the measured values D(f) and/or C(f) against stored reference values $D_{ref}(f)$ or $C_{ref}(f)$, respectively.

21. Apparatus according to Claim 20,
**characterized** in that said evaluating unit calculates the value of A in compliance with the relationship

$$A = (D(f_1) - D(f_2)) / (f_2 - f_1)$$

for determining the alkalinity of an oil in particular, wherein $D(f_i)$ represents the dielectric loss factor at frequency $f_i$, and compares the value A as measured with a stored reference value $A_{ref}$, and outputs the variation or the measured and possibly compensated value A as a direct measure of the alkalinity.

22. Apparatus according to Claim 20 or 21,
**characterized** in that said evaluating unit determines one or more maxima of the dielectric loss factor D(f) as a function of the frequency of the applied a.c. voltage for the determination of the viscosity of a liquid, and determines the viscosity of the analysed liquid on the basis of a comparison of the frequencies against reference frequencies at which maxima occur in a reference liquid.

23. Apparatus according to any of Claims 20 to 22,
**characterized** in that said evaluating unit determines the additive concentration from the magnitude of the maxima.

24. Apparatus according to any of Claims 20 to 23,
**characterized** in that said evaluating unit compares the measured pairs of capacitance/frequency values against stored pairs of capacitance/frequency values, and associates a pH value with these pairs of values.

25. Apparatus according to any of Claims 20 to 24,
**characterized** in that said evaluating unit compares the measured paires of dielectric loss factor/frequency values against stored pairs of dielectric loss factor/frequency values.

26. Apparatus according to any of Claims 20 to 25,
**characterized** in that said evaluating unit outputs an indication and/or control signal for a control variable, e.g. a valve control of a pump or the like, if a predetermined limit of $D(f_r)$ and/or $C(f_r)$ or of data calculated on the basis thereof is exceeded.

## Revendications

1. Détecteur pour l'analyse de liquides, comprenant des électrodes (1, 2) qui présentent une couche électriquement isolante et essentiellement imperméables aux fluides (4), et
une membrane non-conductrice (3) disposée entre lesdites électrodes (1, 2), qui consiste en un oxyde métallique imperméable aux liquides, un système d'oxydes métalliques mixtes, ou une matière isolante

EP 0 182 795 B1

inorganique ou organique, qui a des pores, fissures ou interstices à grande surface, p.e. de l'ordre de 100 m$^2$/g environ, pour permettre le liquide à analyser d'y pénétrer, afin d'obtenir une couche à charge d'espace par voie d'adsorption physique.

2. Détecteur selon la revendication 1,
**caractérisé** en ce que lesdites électrodes (1, 2) consistent essentiellement en aluminium, zirconium, titane, tantale, tungstène et/ou molybdèneou un métal ou alliages de tels métaux similaires.

3. Détecteur selon la revendication 1 ou 2,
**caractérisé** en ce que ladite membrane non-conductrice (3) est essentiellement composée de l'alumine, du nitrure de silicium, de l'oxyde de titane, de $[KAl_2(OH)_2]$ * $[AlSi_3O_{10}]$ ou du polytétrafluoré-thylène.

4. Détecteur selon une quelconque des revendications 1 à 3,
**caractérisé** en ce que lesdites couches électriquement isolantes (4) sur lesdites électrodes (1, 2) consistent essentiellement d'un oxyde de métal non-conducteur, un système d'oxydes mixtes, un nitrure métallique, ou tout autre matière isolante inorganique quelconque.

5. Détecteur selon une quelconque des revendications 1 à 4,
**caractérisé** en ce que lesdites couches (4) sont formées en oxydes amphotères, p.e. $Al_2O_3$ ou rutile, en systèmes d'oxydes mixtures, p.e. le zirconate de titane-aluminium.

6. Détecteur selon une quelconque des revendications 1 à 5,
**caractérisé** en ce que lesdites couches sont produites par oxydation directe de la matière de l'électrode, p.e. dans un processus d'oxydation anodique ou à gaine anodique, pour former l'oxyde de zirconium, l'oxyde de titane, l'oxyde de tantale, l'oxyde de tungstène, l'oxyde de molybdène, l'oxyde d'aluminium ou l'oxyde d'un métal transitoire similaire.

7. Détecteur selon une quelconque des revendications 1 à 6,
**caractérisé** en ce que the l'épaisseur de la couche est d'ordre de 5 $\mu$m à 30 $\mu$m environ.

8. Détecteur selon une quelconque des revendications 1 à 7,
**caractérisé** en ce que lesdites électrodes (1, 2) sont formées du titane pur ou d'un alliage de titane, qui contient p.a. jusqu'à 6% de l'aluminium et jusqu' à 4% de vanadium et éventuellement de cuivre, pendant que lesdites couches d'électrode (4) sont produites de $TiO_2$ et ladite membrane (3) est formée en $Al_2O_3$.

9. Détecteur selon une quelconque des revendications 1 à 7,
**caractérisé** en ce que lesdites couches (4) et ladite membrane (3) consistent essentiellement de la même matière de base chimique, éventuellement à structures différentes.

10. Détecteur selon une quelconque des revendications 1 à 9,
**caractérisé** en ce que le diamètre des pores dans ladite membrane (3) est d'ordre de 0.01 $\mu$m à 1 $\mu$m environ, préférablement entre 0.05 $\mu$m et 0.2 $\mu$m.

11. Détecteur selon une quelconque des revendications 1 à 10,
**caractérisé** en ce
qu'au moins une desdites électrodes (1, 2) est poreuse, et en ce que les parois intérieures poreuses de l'électrode sont recouvertes par une couche non-conductrice imperméable à liquides (4).

12. Détecteur selon une quelconque des revendications 1 à 11,
**caractérisé** en ce que l'épaisseur de la membrane est d'ordre de 10 $\mu$m à 100 $\mu$m, de préférence de 20 $\mu$m à 40 $\mu$m.

13. Détecteur selon une quelconque des revendications 1 à 12,
**caractérisé** en ce que ladite membrane (3) non poreuse est formée d'une matière à structure stratifiée apte à être pénétrée par le liquide à analyser.

14

14. Détecteur selon la revendication 13,
    **caractérisé** en ce que ladite membrane non poreuse est formée de corindon non-poreux, le hydroxylsilicate d'aluminium ou alumine gamma.

15. Détecteur selon la revendication 14,
    **caractérisé** en ce que l'épaisseur de la membrane est d'ordre de 6 à 100 $\mu$m environ, de préférence de 30 à 40 $\mu$m.

16. Détecteur selon une quelconque des revendications 1 à 15,
    **caractérisé** en ce
    que ledit détecteur est renfermé, au moins en partie, par une membrane sélective pour des ions.

17. Détecteur selon la revendication 16,
    **caractérisé** en ce que ladite membrane sélective pour des ions améliore la sélectivité pour les ions d'hydrogène ou hydroxyle ainsi que pour des ions de potassium, du chlorure de sodium, du magnésium ou du calcium.

18. Détecteur selon une quelconque des revendications 1 à 17,
    **caractérisé** en ce
    que lesdites électrodes (1, 2) sont disposées l'une en face de l'autre de façon d'un condensateur à lames.

19. Détecteur selon une quelconque des revendications 1 à 17,
    **caractérisé** en ce qu'une (1) desdites électrodes renferme l'autre électrode (2) en forme d'un cylindre, ladite membrane entourant l'électrode intérieure.

20. Appareil pour mesurer les propriétés d'un liquide, opérant sur un détecteur selon une quelconque des revendications 1 à 19, plongé dans le liquide à analyser,
    **caractérisé** en ce qu'une unité de traitement de signaux et d'évaluation applique une tension alternative auxdites deux électrodes (1,2), la fréquence de ladite tension alternative variant entre 50 Hz et 1 GHz, et mesure le facteur de perte diélectrique $D = D(f)$ et/ou la capacité $C = C(f)$ pour des fréquences différentes, et que ladite unité d'évaluation compare les valeurs mesurées $D(f)$ et/ou $C(f)$ aux valeurs de référence mémorisées $D_{ref}(f)$ ou respectivement $C_{ref}(f)$.

21. Appareil selon la revendication 20,
    **caractérisé** en ce que ladite unité d'évaluation calcule la valeur de A selon la relation

    $$A = (D(f_1) - D(f_2)) / (f_2 - f_1)$$

    pour déterminer l'alcalinité de l'huile en particulier, dans laquelle $D(f_i)$ représent le facteur de perte diélectrique à la fréquence
    $f_i$,
    et compare la valeur A mesurée à une valeur de référence mémorisée $A_{ref}$, et fait sortir la variation ou la valeur A mesurée et éventuellement compensée en tant qu'une mesure directe pour l'alcalinité.

22. Appareil selon la revendication 20 ou 21,
    **caractérisé** en ce que ladite unité d'évaluation détermine un ou plusieurs maximums du facteur de perte diélectrique $D(f)$ en fonction de la fréquence de la tension alternative appliquée pour déterminer la viscosité du liquide, et pour déterminer la viscosité du liquide analysé à base d'un comparaison des fréquences aux fréquences de référence auxquelles se produisent des maximums dans un liquide de référence.

23. Appareil selon une quelconque des revendications 20 à 22,
    **caractérisé** en ce que ladite unité d'évaluation détermine la concentration de l'additif à base de la quantité des maximums.

24. Appareil selon une quelconque des revendications 20 à 23,
    **caractérisé** en ce que ladite unité d'évaluation compare les paires mesurées de valeurs capacité/fréquence aux paires mémorisées de valeurs capacité/fréquence, et les affecte une valeur pH auxdites paires de valeurs.

25. Appareil selon une quelconque des revendications 20 à 24,
**caractérisé** en ce que ladite unité d'évaluation compare les paires mesurées de valeurs de facteur de perte diélectrique/fréquence auxdites paires de valeurs de facteur de perte diélectrique/fréquence mémorisées.

26. Appareil selon une quelconque des revendications 20 à 25,
**caractérisé** en ce que ladite unité d'évaluation faire sortir une indication et/ou un signal de commande pour une variable réglante, p.e. la commande de soupape d'une pompe ou similaire, dès qu'une valeur limite définie de $D(f_r)$ et/ou $C(f_r)$ ou des données calculées à base de cettes limites est dépassée.

# Fig.1

# Fig.2

Fig.3

Fig. 7

$$\frac{D_{400} - D_{100}}{\Delta f} \ [s]$$

Eichkurve für die Alkalität

Fig.4

Fig. 5

Fig.6